# EUROPEAN PATENT APPLICATION

(11) **EP 2 636 378 A1**
(43) Date of publication of application: **11.09.2013**
(21) Application number: 13002903.6
(22) Date of filing: 07.09.2011
(51) Int. Cl.: A61B 17/16, A61C 1/12

(54) **Surgical power tool**

(62) Divisional of application: 11007282.4
(71) Applicant: Stryker Leibinger GmbH & Co. KG, 79111 Freiburg (DE)
(72) Inventor: Schmuck, Manfred, 78570 Mühlheim (DE); Greiner, Karl, 78570 Mühlheim (DE); Knöpfle, Christian, 78166 Donaueschingen (DE)
(74) Representative: Röthinger, Rainer

(57) **Abstract**

A surgical power tool (1) is described. The surgical power tool comprises a head portion (20) having a tool (21) with a tool axis, a shaft (10) which is connected to the head portion for guiding the head portion to the surgical site and an illumination device (40) for providing light to the surgical site. The shaft has a longitudinal axis that is angled with respect to the tool axis.

## Description

### Technical Field

The present disclosure relates to a surgical power tool with a head portion having a tool and a shaft which is connected to the head portion for guiding the head portion to a surgical site.

### Background

Surgical procedures often involve drilling, screwing or other operations at a bone. Surgical power tools are known in the art which support the surgeon when such operations need to be performed.

A surgical power tool is, for example, disclosed in EP 1 836 976 A1. The power tool comprises a drill or similar tool arranged at a distal end of the power tool and a gripping shaft to handle the power tool. The longitudinal axis of the gripping shaft is angled with respect to the drill or other tool.

When the surgeon accomplishes a surgical procedure, he or she has to exert pressure over the gripping shaft of the surgical power tool, so that enough force will arrive at the distally arranged tool. This situation could result in a slip of, for example, the drill at the surgical site, and the patient being treated can get injured. Consequently, it requires a great effort for the surgeon to carry out an accurate drill or cut with a surgical power tool without injuring the patient.

### Summary

There is a need for a surgical power tool which supports the surgeon to carry out an accurate operation at the surgical site and reduces the risk of injuring the patient.

A surgical tool is provided that comprises a head portion having a tool with a tool axis and a shaft which is connected to the head portion for guiding the head portion to a surgical site, wherein the shaft has a longitudinal axis and the tool axis is angled with respect to the longitudinal axis of the shaft. The surgical power tool further comprises an illumination device for providing light to the surgical site.

The surgical power tool may further comprise a handle portion for exerting pressure to the head portion along the tool axis.

The axis of the tool is angled with respect to the shaft of the surgical power tool. This angle may be defined such that the user can easily reach the desired position at the surgical site. Thus, the angle can be between 60 and 120 degrees, for example between 80 and 100 degrees (e.g., approximately 90 degrees). The angle may be adjustable (e.g., mechanically or electrically).

The power tool can be powered by any kind of electric motor. The electric motor can be accommodated in the shaft or the handle portion or can be connected to the surgical power tool by means of a driveshaft. The transfer of the power to the angled tool can be accomplished by any kind of transmission. The power tool can also be powered by pressurized air or in any other manner.

The handle portion may comprise at its proximal end a handle for being gripped by a surgeon. The longitudinal axis of the handle may be substantially parallel with the tool axis. There can be any tolerance (e.g., up to 20 degrees) between the longitudinal axis of the handle and the tool axis. Due to the arrangement of the handle and the tool, pressure can be exerted to the head portion, in particular in the direction of the tool axis. The longitudinal axis of the handle may in particular be coaxial with the tool axis, so that the pressure can be accurately exerted in the direction of the tool axis and therewith to the surgical site.

In an optional aspect, the handle of the handle portion is angled with respect to the shaft, so that pressure can be exerted in a direction toward the tool axis. The resultant force exerted by the surgeon on the handle may be directed towards the tool axis to support the surgical procedure. The angle between the handle and the shaft can be between 60 and 120 degrees, for example between 80 and 100 degrees (e.g., approximately 90 degrees).

According to a further optional aspect, the handle portion is configured to secure the handle portion to the shaft, so that the handle portion is fixed and cannot be moved or rotated with respect to the shaft. This securing can be accomplished in a releasable manner, for example by a clamping element. The clamping element may be arranged at a distal end of the handle portion and can at least partly encompass the shaft. The clamping element can have a C- or U-like shape to accommodate the shaft. Any other shape is feasible as long as the connection can be fixed.

In an exemplary implementation, the connection is such that the handle portion can easily be removed from the shaft (e.g., so that both parts can be individually sterilized). The handle portion can be secured to the shaft by means of a connecting fastener which can be a locking screw, snap-in connection or interlocking connection.

Advantageously, the clamping element is distanced from the head portion, so that the handle portion is not directly connected to the head portion of the surgical power tool. The clamping element may be connected to the shaft close to the head portion to provide a small lever arm defined between the clamping element and the tool along the shaft.

The clamping element may further be designed such that the handle portion can be adjusted relative to the longitudinal axis of the shaft, for example by a guided rail. The clamping element can also comprise some kind of electric contacts, so that information can be transferred from the handle portion to the shaft or the head portion.

The surgical power tool comprises the illumination device for providing light to the surgical site. The light may be guided by an optical cable to the surgical site or a light source can be arranged directly at the head portion. The light may be cool light.

The illumination device can be guided along or inside the handle portion. In particular, the handle portion may be adapted to accommodate at least one part of the illumination device, for example a power supply of the light source, a switch of the light source and/or the light source itself. The illumination device can also be guided along or inside the shaft.

In one realization, the surgical power tool comprises at least one of a mirror and an endoscope which provides a view of the surgical site, in particular the portion of the site illuminated by a illumination device. The mirror or endoscope can be arranged at the head portion, but it can also be arranged at the shaft or the handle portion.

The mirror or endoscope or at least one part of it may be releasably connected to the surgical power tool. The mirror or endoscope may be adjusted, mechanically or electrically, by for example an electric motor. The adjustment may be provided remotely from the shaft or the handle portion.

According to a further optional aspect, the surgical power tool comprises a rinsing device for providing fluid to the surgical site. The rinsing device can be guided at or inside the shaft or handle portion towards the tool. Any system which can provide a constant stream or flash of fluid can be connected to a rinsing inlet of the rinsing device.

In a further realization, a fixture device may be arranged at the head portion, the shaft or the handle portion to hold any parts which are needed to carry out the surgical procedure. In particular, the fixture device may provide a clamping arrangement to hold the needed parts. These parts can comprise a tissue protection sleeve, a fixation module or plate that is be secured to the bone.

### Brief Description of the Drawings

These and other features, aspects and advantages of the present disclosure will become more apparent from the following detailed description taken in conjunction with the accompanying drawings, wherein:
Fig. 1 shows an embodiment of a surgical power tool in a perspective view;
Fig. 2 is an enlarged perspective view of the surgical power tool showing Fig. 1;
Fig. 3 is a further enlarged perspective view of the surgical power tool showing Fig. 1;
Fig. 4 is a perspective view of a further embodiment of a surgical power tool;
Fig. 5 is an enlarged perspective view of the embodiment shown in Fig. 4; and
Fig. 6 is a further enlarged perspective view of the embodiment shown in Fig. 4.

### Detailed Description

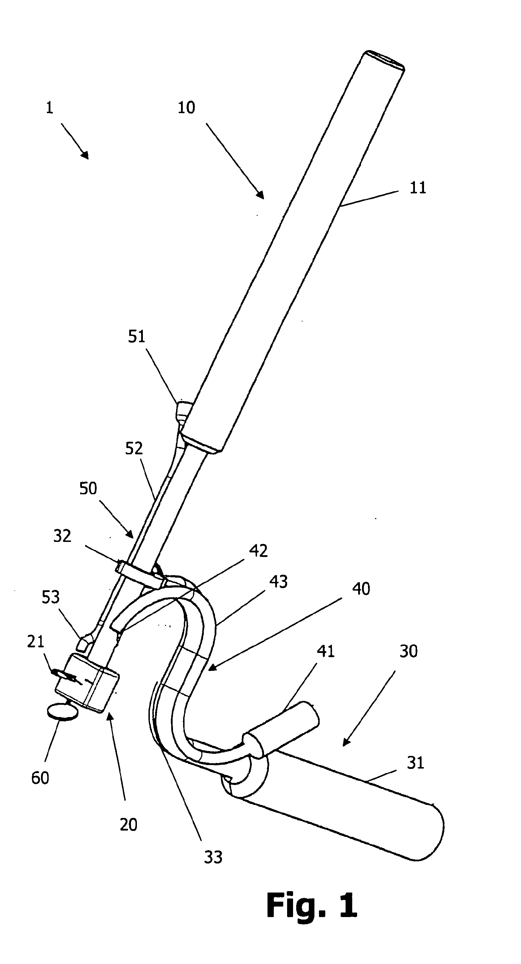

Referring to Fig. 1, there is shown an embodiment of a surgical power tool 1 in the form of a surgical power drill. As shown, the surgical power tool 1 comprises a shaft 10 which is surrounded at its proximal end by a grip 11. The grip 11 extends over approximately at least half of the length of the whole shaft 10, so that a surgeon can easily handle the power tool 1. A head portion 20 is arranged at the distal end of the shaft 10. This head portion 20 carries a tool 21 in the form of a drill to create a bore in the bone at the surgical site. The shaft 10 can comprise components to operate and/or drive the tool 21 of the surgical power tool 1. Moreover, the tool 21 may be removable from the head portion 20.

As illustrated in Fig. 1, the tool 21 as a tool axis that is angled with respect to a longitudinal axis of the shaft 10. In the present embodiment, the angle is 90 degrees and may be adjustable within a predefined range.

The surgical power tool 1 further comprises a handle portion 30 having a handle 31 at its proximal end. The handle 31 is long enough so that at least one hand of the surgeon can easily grasp the handle 31. A longitudinal axis of the handle 31 is parallel to the tool axis of the tool 21.

The handle portion 30 further comprises a linkage 33 and a clamping element 32 at its distal end. The linkage 33 is arranged between the handle 31 and the clamping element 32. The linkage 33 is formed in a bent shape and is rigid, so that application forces can be transferred. Specifically, the linkage 33 has the form of approximately an S to facilitate, for example, intra-oral and/or transbuccal procedures. The linkage 33 is located in a plane defined by a longitudinal axis of the shaft 10 and the longitudinal axis of the handle 31.

The clamping element 32 is arranged at the distal end of the handle portion 30 and is configured to secure the handle portion 30 to the shaft 10. The location of the clamping element 32 is situated on the shaft 10 between the head portion 20 and the grip 11. The clamping element 32 is located spaced apart but near the head portion 20, so that pressure can be exerted to the tool 21 over the clamping element 32 without straddling the linkage 33.

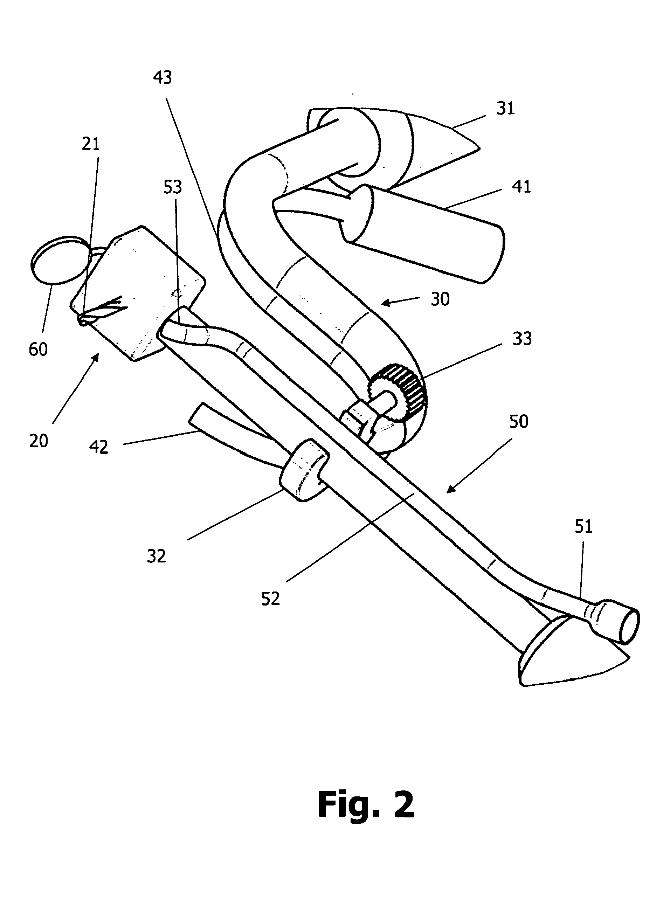

As shown in Fig. 2, the clamping element 32 partly encompasses the shaft 10 and is locked by a connecting fastener 33. The shaft 10 is fixedly clamped by the clamping element 32, so that no movement or rotation of the handle portion 30 relative to the shaft 10 is possible. The connecting fastener 33 is a locking screw which is configured to push one part of the clamping element 32 towards the shaft 10 and therewith provides a frictional engagement. Thus, handle 30 and the shaft 10 are releasably fixed to each other.

A head of the locking screw 34 is large enough so that the surgeon can easily secure and release the locking screw 34. Due to the straight shape of the shaft 10, the clamping element 32 can be axially moved along the shaft 10 to any desired position when the locking screw 34 is released. The handle 31 can also be removed from the shaft 10 for cleaning and sterilization activities.

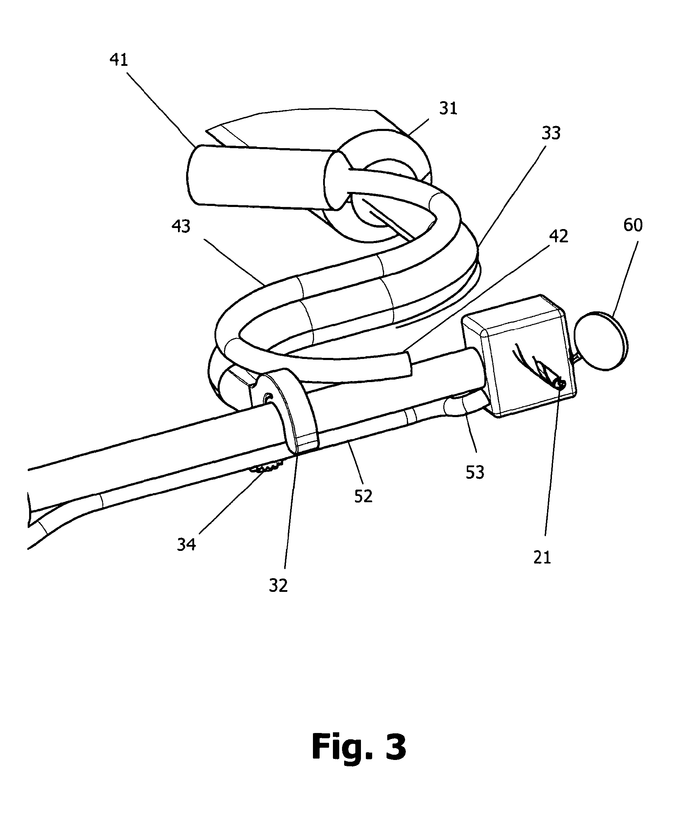

As illustrated in Figs. 2 and 3, an illumination device 40 comprising a light inlet 41, an optical cable 43 and a light outlet 42 is (mainly) arranged along the linkage 33 of the handle portion 30. The light inlet 41 comprises a connector (not shown) to receive a light unit which generates a beam of cool light. The light outlet 42 is directed toward the tool 21 to illuminate the surgical site, wherein the light outlet 42 can be movable to position the light outlet 42 such that the surgeon is not bothered.

The surgical power tool 1 further comprises a rinsing device 50 having a rinsing inlet 51, a tube 52 and a rinsing outlet 53. The rinsing inlet 51 is configured to be coupled to any device capable of providing a rinsing fluid. The tube 52 leads the fluid from the rinsing inlet 51 to the rinsing outlet 53. The rinsing outlet 53 can be selectively positioned by the surgeon such that the fluid is directed towards the surgical site. The rinsing device 50 is guided along and connected to the part of the shaft 10 which is not encompassed by the grip 11.

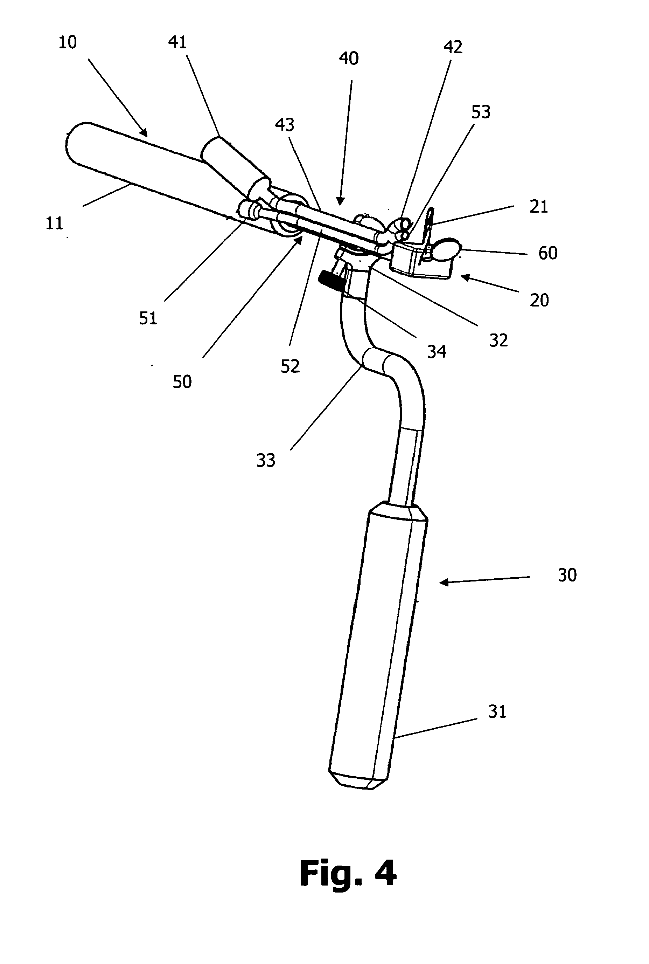

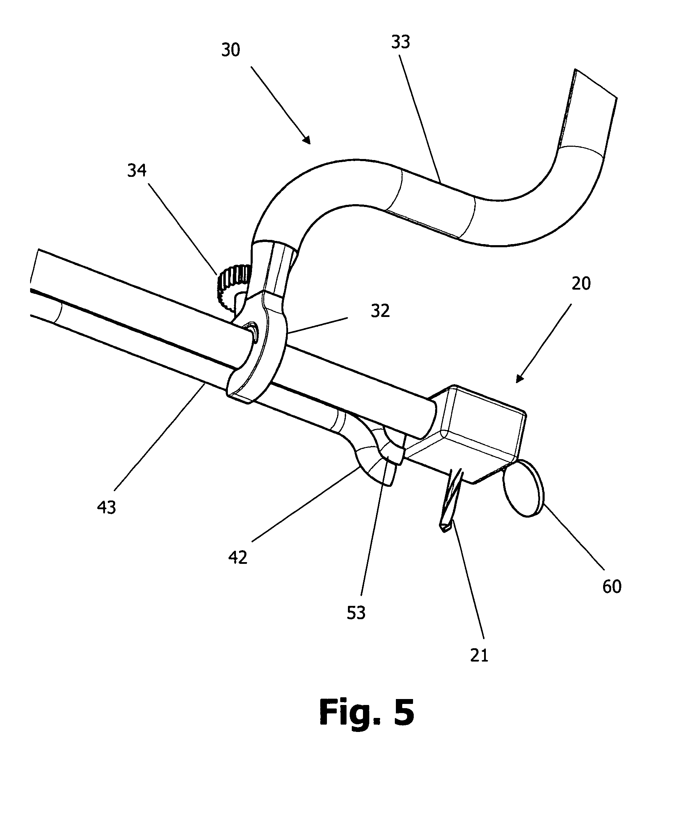

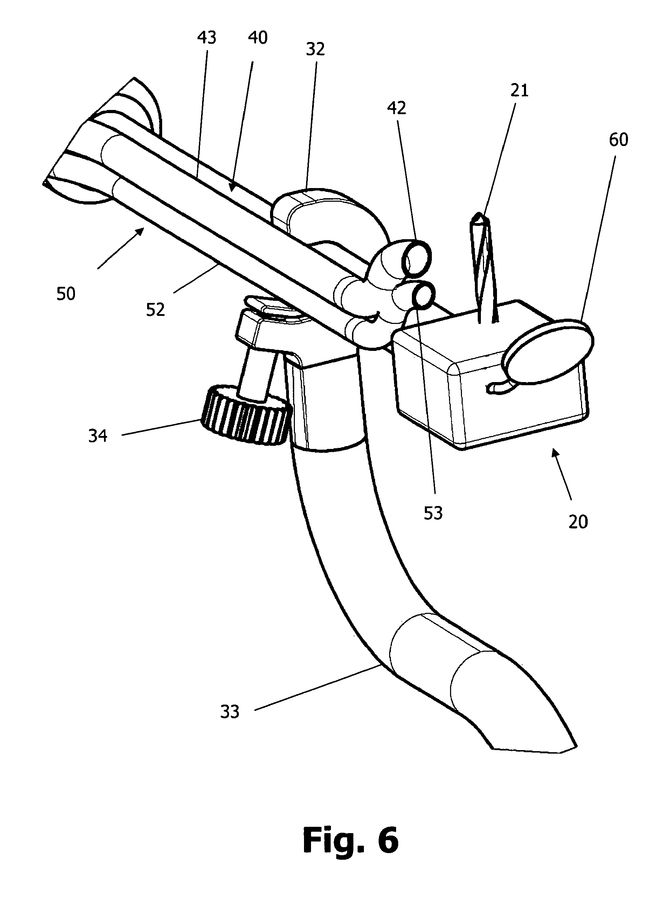

A mirror 60 is arranged at the head portion 20 opposite of the shaft 10, so that the surgeon operating the surgical power tool 1 can view the surgical site through this mirror 60. The mirror 60 can be adjusted as needed by the surgeon. In addition or as an alternative to the mirror 60, an endoscope may be used to provide a view of the surgical site. The endoscope may be attached to the surgical power tool 1 in a similar manner as (e.g., instead of) the illumination device 40 and the rinsing device 50. Figs. 4 to 6 show a further embodiment of a surgical power tool 1. The difference between this embodiment and the aforementioned embodiment is that the illumination device 40 is now arranged at the shaft 10 next to the rinsing device 50. All other parts and features correspond to the aforementioned embodiment.

As has become apparent from the embodiments, the shaft for guiding the head portion to the surgical site and the handle portion to exert pressure to the tool are separated from each other, so that the surgeon can accurately guide the head portion to the desired position with one hand and can further exert pressure to the handle portion with the other hand.

The surgical power tool described in the above embodiments has a tool in the form of a drill. It will be appreciated that the tool could alternatively be realized by a screw driver blade or in any other manner.

While the present disclosure has been described with respect to particular embodiments, those skilled in the art will recognize that the present invention is not limited to the specific embodiments described and illustrated herein. It is to be understood that the disclosure is only illustrative. Accordingly, it is intended that the invention be limited only by the scope of the claims attended hereto.

## Claims

1. A surgical power tool (1) comprising
- a head portion (20) having a tool (21) with a tool axis,
- a shaft (10) which is connected to the head portion (20) for guiding the head portion (20) to a surgical site, the shaft (10) having a longitudinal axis and the tool axis being angled with respect to the longitudinal axis of the shaft (10), and
- an illumination device (40) for providing light to the surgical site.

2. The surgical power tool according to claim 1, further comprising a handle portion (30) for exerting pressure to the head portion (20) along the tool axis.

3. The surgical power tool according to claim 1 or 2, wherein the illumination device (40) is guided along or inside the shaft (10).

4. The surgical power tool according to claim 2, wherein the illumination device (40) is guided along or inside the handle portion (30).

5. The surgical power tool according to one of the preceding claims, wherein the handle portion (30) or the shaft (10) is adapted to accommodate at least one part of the illumination device (40).

6. The surgical power tool according to one of the preceding claims, wherein the illumination device (40) comprises an optical cable (43) for guiding the light to the surgical site.

7. The surgical power tool according to one of the preceding claims, wherein the illumination device (40) comprises a light inlet (41) and a light outlet (42), wherein the light inlet (41) includes a connector for receiving a light source and the light outlet (40) is directed toward the tool (21) to illuminate the surgical site.

8. The surgical power tool according to claim 7, wherein the light outlet (40) is movable for positioning the light outlet (40).

9. The surgical power tool according to one of the preceding claims, wherein a light source is arranged at the head portion (20).

10. The surgical power tool according to one of the preceding claims, wherein the light is cool light.

11. The surgical power tool according to one of the preceding claims, wherein the tool axis is angled with an angle of 60 to 120 degrees with respect to the longitudinal axis of the shaft (10).

12. The surgical power tool according to one of the preceding claims, wherein the handle portion (30) is configured to be releasably secured to the shaft (10).

13. The surgical power tool according to one of the preceding claims, wherein the surgical power tool (1) further comprises at least one of a mirror (60) and an endoscope configured to provide a view of the surgical site.

14. The surgical power tool according to claim 13, wherein the mirror (60) and/or endoscope are/is arranged at the head portion (20), the shaft (10) or the handle portion (30).

15. The surgical power tool according to one of the preceding claims, wherein the surgical power tool (1) further comprises a rinsing device (50) for providing fluid to the surgical site, wherein the rinsing device (50) is arranged at least partly along at least one of the shaft (10) and the handle portion (30).
